(19) Europäisches Patentamt
European Patent Office
Office européen des brevets

(11) EP 1 017 680 B1

(12) FASCICULE DE BREVET EUROPEEN

(45) Date de publication et mention de la délivrance du brevet:
**07.02.2007 Bulletin 2007/06**

(21) Numéro de dépôt: **98945362.6**

(22) Date de dépôt: **23.09.1998**

(51) Int Cl.:
***C07D 233/52*** (2006.01) ***C07D 233/50*** (2006.01)
***C07D 239/18*** (2006.01) ***C07D 239/14*** (2006.01)
***C07D 401/12*** (2006.01) ***C07D 407/12*** (2006.01)
***C07C 69/76*** (2006.01) ***C07C 69/003*** (2006.01)
***C07C 65/40*** (2006.01) ***C07C 235/84*** (2006.01)
***C07C 271/22*** (2006.01) ***A61K 31/415*** (2006.01)
***A61K 31/505*** (2006.01)

(86) Numéro de dépôt international:
**PCT/FR1998/002038**

(87) Numéro de publication internationale:
**WO 1999/015506 (01.04.1999 Gazette 1999/13)**

# (54) COMPOSES TRICYCLIQUES, LEUR PROCEDE DE PREPARATION ET LES INTERMEDIAIRES DE CE PROCEDE, LEUR APPLICATION A TITRE DE MEDICAMENTS ET LES COMPOSITIONS PHARMACEUTIQUES LES RENFERMANT

DREIRINGIGE VERBINDUNGEN, VERFAHREN ZU IHRER HERSTELLUNG UND ZWISCHENPRODUKTE DIESES VERFAHREN, IHRE ANWENDUNG ALS ARZNEIMITTEL UND SIE ENTHALTENDE PHARMAZEUTISCHE ZUSAMMENSETZUNGEN

TRICYCLIC COMPOUNDS, PREPARATION METHOD AND SAID METHOD INTERMEDIATES, APPLICATION AS MEDICINES AND PHARMACEUTICAL COMPOSITIONS CONTAINING SAME

(84) Etats contractants désignés:
**AT BE CH CY DE DK ES FI FR GB GR IE IT LI LU NL PT SE**

(30) Priorité: **24.09.1997 FR 9711858**

(43) Date de publication de la demande:
**12.07.2000 Bulletin 2000/28**

(73) Titulaires:
- **Aventis Pharma S.A.**
  **92160 Antony (FR)**
- **GENENTECH, INC.**
  **South San Francisco, CA 94080-4990 (US)**

(72) Inventeurs:
- **CARNIATO, Denis**
  **F-91460 Marcoussis (FR)**
- **GADEK, Thomas, R.**
  **Oakland, CA 94611 (US)**
- **GOURVEST, Jean-François**
  **F-77410 Claye-Souilly (FR)**
- **KNOLLE, Jochen**
  **D-65830 Kriftel (DE)**
- **McDOWELL, Robert**
  **San Francisco, CA 94114 (US)**
- **PEYMAN, Anuschirwan**
  **D-65779 Kelkheim (DE)**

(56) Documents cités:
**EP-A- 0 729 933**
**WO-A-88/08842**
**WO-A-96/06087**
**WO-A-97/34865**
**FR-A- 2 446 285**

- **CHEMICAL ABSTRACTS, vol. 67, no. 11, 11 septembre 1967 Columbus, Ohio, US; abstract no. 54032, XP002069323 & JP 42 005537 B -& JP 42 005537 B (SHIONOGI AND CO., LTD.)**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 017 680 B1

## Description

La présente invention concerne de nouveaux composés tricycliques, leur procédé de préparation et les intermédiaires de ce procédé, leur application à titre de médicaments et les compositions pharmaceutiques les renfermant.

WO97/34865 décrit des dérivés tricycliques ayant une activité inhibitrice de la résorption osseuse. Les composés selon l'invention diffèrent de cet art antérieur au niveau de la nature des substituants $R'_1$.

La présente invention a pour objet les composés de formule générale (I') :

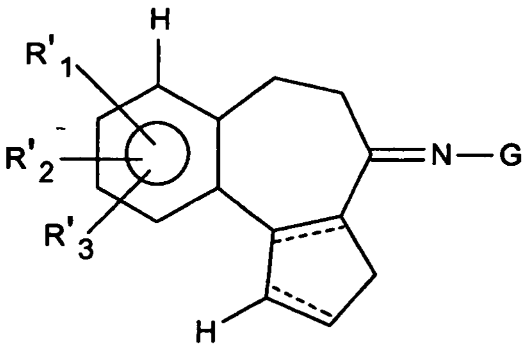

(I')

dans laquelle $R'_1$ représente un groupement
$-CONH-[A]-[B]-COR_6$, -[A]- représentant un radical bivalent
$-CH_2-CH_2-$, substitué par le groupement (Z') ou non substitué,
[B] représentant une simple liaison,
(Z') représente un atome d'hydrogène, un groupement
$-NH-CO_2-Rc$ ou Rc,
Rc représente un atome d'hydrogène, un radical $(CH_2)_{0-3}$-Ar dans lequel Ar représente un groupement aryle carbocyclique
renfermant de 6 à 18 atomes de carbone, un radical $(CH_2)_{0-3}$-Het dans lequel Het représente un radical dérivé d'un hétérocycle aromatique ou non aromatique, saturé ou non saturé,
comportant de 1 à 9 atomes de carbone et de 1 à 5
hétéroatomes choisis parmi les atomes d'oxygène, d'azote ou de soufre, un radical $(CH_2)_{0-3}$-Alk dans lequel Alk représente radical dérivé d'un hydrocarbure, non aromatique, linéaire, ramifié ou cyclique, saturé ou insaturé, et comportant de 1 à 12 atomes de carbone, le radical Ar étant non substitué ou bien substitué par $-O-CH_2-O-$ lorsque Ar est phényle,

- $R_6$ représente un radical hydroxyle,
- $R'_2$ et $R'_3$ identiques ou différents représentent un atome d'hydrogène ou un radical O-Me,
- G représente,
  soit un radical de formule G1

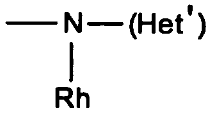

dans lequel Rh est un atome d'hydrogène ou un groupement (Alk) tel que défini précédemment et (Het') est un hétérocycle de formule générale :

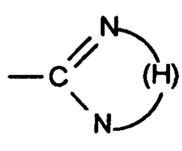

dans lequel (H) forme, avec le motif N=C-NH-, le reste d'un hétérocycle non aromatique, mono ou bicyclique, non saturé, comportant de 1 à 9 atomes de carbone et de 2 à 5 hétéroatomes choisis parmi les atomes d'oxygène, d'azote et de soufre, ce radical pouvant être substitué ou non substitué,

- soit un radical -NRh-C(=X)-NHRc (radical G2), dans lequel X est un atome de soufre, d'oxygène ou NH, Rh et Rc sont tels que définis précédemment,

les traits en pointillés représentent une éventuelle seconde liaison, ainsi que les sels d'addition avec les acides et les bases.
$R'_1$, $R'_2$ et $R'_3$ étant respectivement en position 8, 9 ou 10 du tricycle.

Par composé de formule (I') on désigne tous les isomères géométriques et les stéréoisomères possibles pris individuellement ou en mélange.

Lorsque Rc représente un groupement $(CH_2)_{0-3}$-Ar, $(CH_2)_{0-3}$-Het, $(CH_2)_{0-3}$-Alk, $(CH_2)_{0-3}$ représente soit une simple liaison dans le cas de $(CH_2)_0$, soit les radicaux $-CH_2-$, $-(CH_2)_2-$ ou $-(CH_2)_3-$.

Par le terme (Ar) représentant un groupement aryle carbocyclique renfermant de 6 à 18 atomes de carbone, on entend un radical dérivé d'un hydrocarbure cyclique aromatique tel que le radical phényle, naphtyle, phénanthrènyl ou bien un radical dérivé d'un hydrocarbure bicyclique ou tricyclique condensé comportant un cycle benzénique tel que indanyle, indényle, dihydronaphtyle, tétrahydronaphtyle ou fluorènyl. La jonction s'effectue au niveau du cycle benzénique. Il s'agit de préférence du phényle.

Par le terme (Het) représentant un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote et de soufre, on désigne notamment :

- les radicaux monocyclique hétérocycliques, par exemple les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle,
- les cycles condensés hétérocycliques, par exemple le benzofurannyle, le benzothiényle, le benzimidazolyle, le benzothiazolyle, le naphto[2,3-b]thiényle, le thianthrényle, l'isobenzofurannyle, le chroményle, le xanthényle, le phénoxathiinyle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'isoindolinyle, l'imidazopyridyle, l'imidazopyrimidinyle ou encore les systèmes polycycliques condensés constitués de monocycliques hétérocycliques tels que définis ci-dessus comme par exemple le furo[2,3-b]pyrrole ou le thiéno [2,3-b]furanne,
- ou les hétérocycles saturés tels que pyrrolidine, pipéridine, morpholine.

Ce terme (Het) englobe par ailleurs les valeurs de (Het') telles que définies précédemment.

Par le terme (Alk) représentant un radical dérivé d'un hydrocarbure, non aromatique, linéaire, ramifié ou cyclique, saturé ou insaturé, on désigne dans le cas des hydrocarbures acycliques les radicaux alkyles tels que méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle, les radicaux alkényles tels que vinyle, propényle, isopropényle, allyle, 2-méthylallyle, butényle ou isobutényle, ou les radicaux alkynyles tels que éthynyle, propynyle, propargyle, butynyle ou isobutynyle, et dans le cas des radicaux cycliques, les radicaux cycloalkyles, tels que cyclopropyle, cyclobutyle, cyclopentyle, cyclohexyle ou adamantyle.

Lorsque G est un radical de formule G1

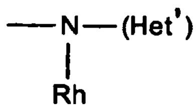

et (Het') est un hétérocycle de formule générale :

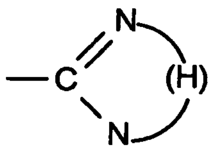

dans lequel (H) forme, avec le motif N=C-NH-, un hétérocycle non aromatique, mono ou bicyclique, non saturé, comportant de 1 à 9 atomes de carbone et de 2 à 5 hétéroatomes choisis parmi les atomes d'oxygène, d'azote et de soufre, ce radical pouvant être substitué ou non substitué, G1 représente notamment des hétérocycles suivants :

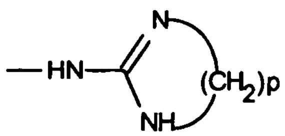 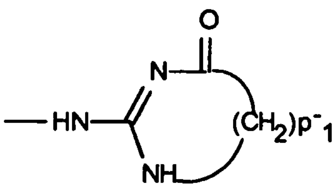 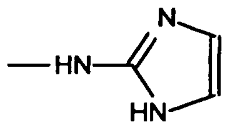

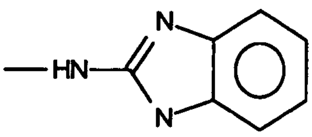 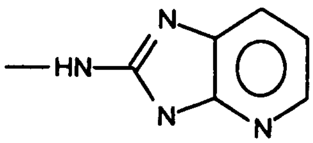 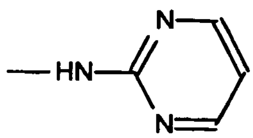

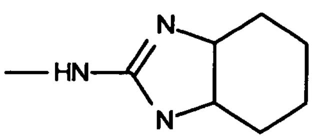

dans lesquels p représente un entier de 1 à 4.

- Lorsque G est un radical -NRh-C(=X)-NHRc (radical G2), dans lequel X est un atome de soufre, d'oxygène ou NH, Rh et Rc sont tels que définis précédemment. Il s'agit notamment des groupements -NB-C(=NB)-$NB_2$, -NB-C(=O)-$NB_2$ ou -NH-C(=S)-$NH_2$,-NB-C(=NB)-$HBCB_2$-Ar tel que -NB-C(=HB)-$HBCS_2Ph$, -NH-C(=NH)-$NHCH_2$-Het, -NH-C(=NH)-$NHCH_2$-Het', -NH-C(=NH)-NH-Alk tel que-NH-C(=NH)-$NHCH_3$, les groupements Ar, Het, Het' ou Alk étant substitués ou non substitués.

Les substituants éventuels des radicaux (Alk), (Ar), (Het), (Het') formant un hétérocycle, sont de préférence les radicaux suivants :

- halogène : fluor, chlore, brome, iode,
- alkyle, alkényle, alkynyle renfermant de 1 à 12 atomes de carbone tel que méthyle, éthyle, propyle, isopropyle, butyle, isobutyle, tert-butyle, vinyl ou allenyl. Ces radicaux étant eux mêmes éventuellement substitués par un ou plusieurs atome d'halogène, par exemple le fluor tel que le trifluorométhyle.

- oxo, cyano, nitro, formyl, carboxy et carboxyalkyl renfermant de 1 à 6 atomes de carbone, carboxamide,
- alkoxy renfermant de 1 à 12 atomes de carbone tel que méthoxy, éthoxy, propyloxy, isopropyloxy, butyloxy,
- alkylthio renfermant de 1 à 12 atomes de carbone tel que méthylthio, éthylthio, propylthio, isopropylthio, butylthio,
- amino, alkylamino renfermant de 1 à 12 atomes de carbone tel que méthylamino ou éthylamino, dialkylamino renfermant de 2 à 24 atomes de carbone tel que diméthylamino, diéthylamino, méthyléthylamino, chacun de ces radicaux dialkylamino étant éventuellement sous forme oxydée,
- aminoalkyle renfermant de 1 à 12 atomes de carbone tel que aminométhyle ou aminoéthyle,
- dialkylaminoalkyle renfermant de 3 à 25 atomes de carbone tel que diméthylamino méthyle ou éthyle,
- dialkylaminoalkyloxy renfermant de 3 à 25 atomes de carbone tel que diméthylaminoéthyloxy,
- hydroxyle éventuellement acylé renfermant de 1 à 12 atomes de carbone, par exemple acétoxy,
- acyle renfermant de 1 à 12 atomes de carbone tels que formyle, acétyle, propionyle, butyryle, isobutyryle, valéryle, isovaléryle, succinyle, pivaloyle benzoyle éventuellement substitué par exemple par un atome de chlore, d'iode ou de fluor. On peut citer les radicaux chloroacétyle, dichloroacétyle, trichloroacétyle, bromoacétyle ou trifluoroacétyle,
- aryle carbocyclique ou hétérocyclique tel que phényle, furyle, thiènyle, pyridinyle ou aralkyle tel que benzyle, ces radicaux étant eux-mêmes éventuellement substitués par des radicaux halogène, alkyle, alkoxy, alkylthio, amino alkyle ou dialkylamino indiqués ci-dessus.

Lorsque Ar représente un phényle, celui-ci peut être substitué par un groupement O-$(CRdRe)_n$-O, n étant un entier de 1 à 5, Rd et Re indépendamment l'un de l'autre représentent un atome d'hydrogène, un radical alkyle renfermant de 1 à 6 atomes de carbone, ou un radical phényle. Il s'agit notamment des radicaux -O-$CH_2$-O-, -O-$C(Me)_2$-O-, -O-C$(Ph)_2$-O-, -O-C$(CH_3)$ (Ph) -O-.

Bien entendu, un ou plusieurs substituants, identiques ou différents, peuvent être présents. Dans le cas de (Het) les substituants peuvent être au niveau du groupement NH ou de l'atome de carbone.

Il est bien entendu que lorsque $R'_1$, $R'_2$, $R'_3$, $R_6$, $R_c$ représentent un groupement alkyle, aryle ou hétérocycle tels que définis ci-dessus, ils peuvent être identiques ou différents indépendamment les uns des autres.

L'invention s'étend naturellement aux sels des composés de formule (I'), comme par exemple les sels formés, lorsque les composés de formule (I') comportent une fonction amino ou amino guanidine, avec les acides chlorhydrique, bromhydrique, nitrique, sulfurique, phosphorique, acétique, trifluoroacétique, formique, propionique, benzoique, maléique, fumarique, succinique, tartrique, citrique, oxalique, glyoxylique, aspartique, alcanesulfoniques tels que les acides méthane ou éthanesulfonique, arènesulfoniques, tels que les acides benzène ou paratoluène sulfonique et arylcarboxylique, ou lorsque les composés de formule (I') comportent une fonction acide, avec les sels des métaux alcalins ou alcalino terreux ou d'ammonium éventuellement substitué.

L'invention s'étend également aux esters des composés de formule (I').

Dans un premier groupe préféré l'invention a pour objet les composés de formule générale (I') telle que définie précédemment dans laquelle $R'_1$ représente un groupement -CONH-CH(Z')-$CH_2CO_2H$ ou -CONH-$CH_2$-CE(Z')-$CO_2H$, ainsi que les sels d'addition avec les acides, les bases et les esters.

Dans un deuxième groupe préféré, l'invention a pour objet les composés de formule générale (I') telle que définie précédemment, dans laquelle (Z') est un groupement aryle ou hétéroaryle non substitué, ou un groupement phényle substitué par -O-$CH_2$-O-, ainsi que les sels d'addition avec les acides, les bases et les esters.

Dans un troisième groupe préféré, l'invention a pour objet les composés de formule générale (I') telle que définie précédemment, dans laquelle G est un groupement G2 de formule -NE-C(=NH)-NERc, Rc étant tel que défini précédemment, ainsi que les sels d'addition avec les acides, les bases et les esters.

Dans un quatrième groupe préféré, l'invention a pour objet les composés de formule générale (I') telle que définie précédemment, dans laquelle G est un groupement G2 de formule NB-C(=NH)-$NH_2$, ainsi que les sels d'addition avec les acides, les bases et les esters.

Dans un cinquième groupe préféré, l'invention a pour objet les composés de formule générale (I') telle que définie précédemment, dans laquelle G est un groupement -NH-(Het') tel que défini précédemment.

Dans un sixième groupe préféré, l'invention a pour objet les composés de formule générale (I') telle que définie précédemment, dans laquelle G est le groupement

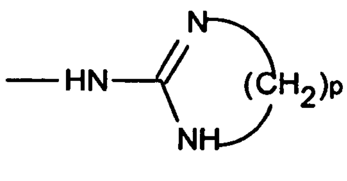

p étant un entier égal à 2, 3 ou 4, ainsi que les sels d'addition avec les acides, les bases et les esters.

Dans un septième groupe préféré, l'invention a pour objet les composés de formule (I') telle que définie précédemment dont les noms suivent:

- 3-[[[4-[(4,5-dihydro-1H-imidazol-2-yl)hydrazono]-9,10-diméthoxy-1,2,3,4,5,6-hexahydro-8-benz[e]azulenyl]-carbonyl]amino]-N-[(phénylméthoxy)carbonyl]-DL-alanine
- 3-[[[9,10-diméthoxy-1,2,3,4,5,6-hexahydro-4-[(1,4,5,6-tétrahydropyrimidin-2-yl)]-hydrazono]-8-benz[e] azulenyl] carbonyl]amino]-N-[(phénylméthoxy)carbonyl]-DL-alanine
- Acide bêta-[[[4-[(4,5-dihydro-1H-imidazol-2-yl) hydrazono]-1,2,3,4,5,6-hexahydro-8-benz[e]azulenyl]carbonyl]-amino]-3-pyridine-3-yl-propanoique
- Acide bêta-[[[4([(4,5-dihydro-1H-imidazol-2-yl) hydrazono]-(1,2,3,4,5,6-hexahydro-4-oxo-8-benz[e]azulenyl]carbonyl]-amino]-3-(1,3-benzodioxole-5-yl-propanoïque.

L'invention a également pour objet un procédé de préparation des composés de formule générale (I') comprenant les étapes suivantes :

a) action d'un agent activant de la fonction alcool puis réaction de carbonylation des composés de formule (II) :

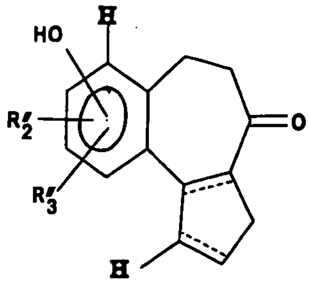

(II)

dans laquelle $R'_2$, $R'_3$ sont tels que définis précédemment, à l'exception de la valeur hydroxyle, afin d'obtenir l'ester de formule (IIIa) :

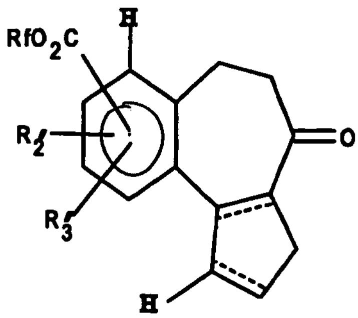

(IIIa)

Rf étant un radical alkyle renfermant de 1 à 4 atomes de carbone,

b) saponification de l'ester de formule (IIIa) afin d'obtenir l'acide correspondant de formule (IIIb) :

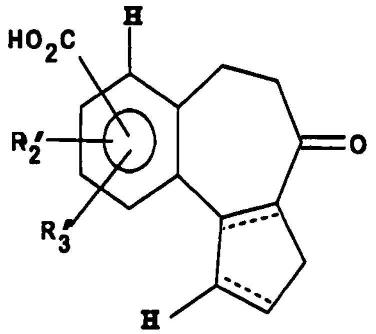

(IIIb)

c) réaction d'amidification de l'acide de formule (IIIb) par action d'un composé de formule (F1), le cas échéant sous forme de sel :

$B_2N$-[A]-[B]-$COR_6$ (F1)

[A], [B] et $R_6$ étant tels que définis précédemment, [A] ou [B] pouvant également représenter le groupement -CH-NHP, P étant un groupement protecteur de la fonction amine, afin d'obtenir un composé de formule (IIIc) :

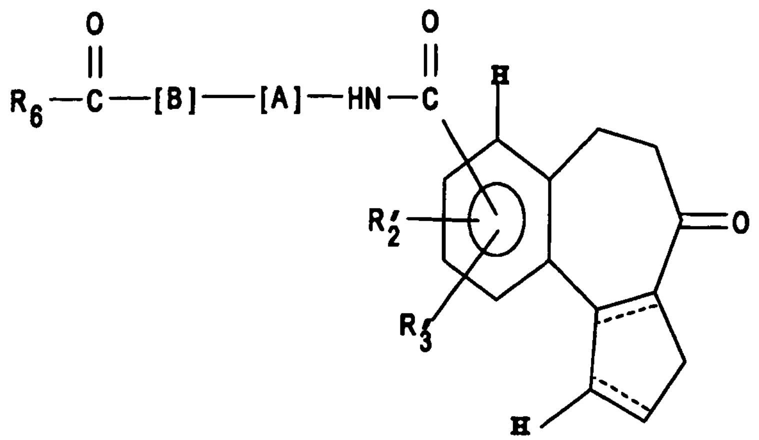

(IIIc)

d) action sur le composé de formule (IIIc) d'un composé de formule (F2) :

G-$NH_2$ (F2)

G étant tel que défini précédemment, afin d'obtenir un composé de formule (I'),
e) composé de formule (I') que l'on soumet le cas échéant dans un ordre approprié :

- action d'une base ou d'un acide afin de cliver l'ester et obtenir l'acide correspondant,
- à l'action d'un réactif de déalkylation,
- à l'action d'un agent de déprotection de la fonction NH-P en bêta de CO-$R_6$ lorsque [A] ou [B] représente le groupe CH-NHP,
- à la formation du groupement NE-$CO_2R_c$ à partir de l'amine correspondante,
- à l'action d'un acide ou d'une base afin d'obtenir les sels correspondants ou
- à l'action d'un acide ou d'une base afin d'obtenir les sels correspondants ou à l'action d'un agent d'estérification afin d'obtenir les esters correspondants.

La réaction de carbonylation s'effectue notamment selon la méthode décrite par P. Prince, D. Richard et D. Gandour dans Synlett (1991) 405, et dans "Palladium Reagents in Organic Synthesis" ed. Hech R.F. Academic Press N.Y. Chap. 8 (1995).

L'activation de l'alcool peut s'effectuer notamment à l'aide de l'anhydride triflique de formule $(CF_3SO_2)_2O$ en présence d'une base telle que la pyridine afin de former le triflate correspondant de formule $(OSO_2CF_3)$.

L'action du composé de formule $R_2N$-[A]-[B]-$COR_6$ (F2) est effectuée de préférence en milieu basique, dans un solvant tel que le diméthylformamide.

L'action de $NH_2$-G (F2) s'effectue, soit sans solvant, soit dans un solvant alcoolique tel que l'éthanol, l'isopropanol ou le butanol. Le synthon $NB_2$-G est éventuellement utilisé sous la forme d'un sel tel que le chlorhydrate ou le bromhydrate.

La réaction de saponification de la fonction ester s'effectue par exemple par action d'une base alcaline telle que la soude ou la potasse dans le tétrahydrofuranne ou un alcool inférieur tel que le méthanol ou l'éthanol. On peut également cliver l'ester en milieu acide selon les méthodes connues de l'homme du métier.

La réaction de déalkylation permettant d'accéder aux produits de formule (I') avec $R'_2$, ou $R'_3$ représentant des hydroxyles s'effectue en présence de chlorure d'aluminium ou de tribromure de bore.

La fonctionnalisation de $NH_2$, [A] ou [B] représentant $CE(NH_2)$ ou $CR(NB_2.Bcl)$, s'effectue selon les méthodes classiques connues en chimie organique.

La formation de $BECO_2R_c$ à partir de l'amine correspondante s'effectue de préférence par action de $R_cOH$ selon la méthode décrite dans J. Org. Chem., 61, 3929-3934 après avoir fait agir au préalable le triphosgène en présence de bicarbonate de sodium afin d'obtenir intermédiairement l'isocyanate.

Les réactions de salification peuvent être effectuées dans des conditions usuelles. On opère par exemple, pour salifier le groupement terminal $CO_2H$ de $R'_1$, en présence d'un sel de sodium tel que le carbonate de sodium ou le carbonate acide de sodium ou de potassium.

De même, la salification de l'amine ou de l'aminoguanidine que peuvent représenter G, par un acide, est réalisée dans les conditions usuelles. On opère par exemple avec l'acide chlorhydrique, par exemple en solution éthérée.

L'estérification éventuelle des produits est effectuée dans les conditions classiques connues de l'homme du métier.

On opère en général en faisant réagir l'acide de formule (I) ou un dérivé fonctionnel avec un réactif capable d'introduire le groupe ester dont une liste non exhaustive figure ci-dessus dans la définition de $R_6$.

Les produits de formule générale (F1) ou (F2) sont connus ou préparés selon les méthodes connues de l'homme du métier.

On peut également inverser l'ordre de greffage des différents réactifs, à savoir on soumet le composé de formule (II) à l'action d''un composé de formule F2 afin d'obtenir intermédiairement le produit de formule (IIId) :

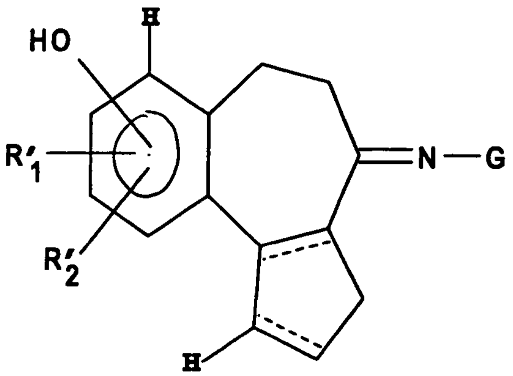

(IIId)

qui est mis en oeuvre ensuite dans les réactions telles que décrites aux étapes a), b), c) et le cas échéant e) afin d'obtenir les produits de formule (I').

Dans ce cas, il faudra le cas échéant, prévoir une protection du groupement G du produit de formule (IIId) puis, après introduction de (F1) ou de (F2), une déprotection selon les méthodes connues de l'homme du métier (T.W. GREENE Protective Groups in Organic Synthesis. John Wiley and Sons Inc. 1991).

La réaction de déprotection du groupement NH-P en bêta de $CO-R_6$, [A] ou [B] représentant le groupe CH-NHP, s'effectue également selon les méthodes connues de l'homme du métier, notamment lorsque P représente le groupe $CO_2tBu$, par une réaction de décarboxylation telle que par exemple par action de l'acide chlorhydrique.

L'os est constamment soumis à un processus dynamique qui inclut la résorption osseuse et la formation osseuse. Ces processus sont médiés via des cellules spécialisées. La formation osseuse est le résultat du dépôt d'une

matrice minérale par les ostéoblastes et la résorption osseuse est le résultat de la dissolution de cette matrice osseuse par les ostéoclastes. L'ostéoporose est caractérisée par une perte sèche de cette matrice osseuse. Un ostéoclaste mature activé résorbe l'os après adhésion à la matrice osseuse via la sécrétion d'enzymes protéolytiques, et de protons à l'intérieur de la zone d'adhésion, aboutissant à des dépressions ou des creusements de la surface de l'os qui apparaissent au moment où l'ostéoclaste se détache de l'os.

Les composés de formule (I') ainsi que leurs sels d'addition pharmaceutiquement acceptables présentent d'intéressantes propriétés pharmacologiques. Ces composés inhibent la résorption osseuse qui est médiée via les ostéoclastes.

Les composés de l'invention sont ainsi utiles dans le traitement de maladies provoquées par la perte de la matrice osseuse, notamment, l'ostéoporose, l'hypercalcémie de malignité, l'ostéopénie due aux métastases osseuses, les paro-dontites, l'hyperparathyroidisme, les érosions periarticulaires dans l'arthrite rhumatoïde, la maladie de paget, l'ostéopénie induite par l'immobilisation, les traitements par les glucocorticoïdes ou les déficiences d'hormones sexuelles mâles ou femelles.

Ils peuvent également être utilisés pour le traitement des désordres inflammatoires, cancéreux et cardiovasculaires incluant l'athérosclérose, la resténose.

Ils peuvent enfin être utilisés comme inhibiteurs de l'angiogenèse et donc dans le traitement des tumeurs, par inhibition de leur néovascularisation, des rétinopathies diabétiques et des néphropathies.

Des études récentes ont montré que la fixation de l'ostéoclaste à l'os est médié par des récepteurs : les intégrines.

Les intégrines sont une superfamille de récepteurs médiant les processus d'adhésion cellule/cellule et plus particulièrement cellule/matrice, incluant notamment $\alpha$2b$\beta$3 comme récepteur des plaquettes sanguines (fibrinogène) et $\alpha$v$\beta$3 comme récepteur de la vitronectine, des sialoprotéines osseuses comme l'ostéopontine et la thrombospondine.

Ces récepteurs qui sont des hétérodimères protéïques composés de deux sous unités $\alpha$ et $\beta$, possèdent des sites de fixation d'ions divalents comme le $Ca^{2+}$ notamment et un site de reconnaissance de leur ligand prédéfini par la qualité de leurs sous unités.

Le récepteur $\alpha$v$\beta$3 est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés selon l'invention.

Les récepteurs $\alpha$v$\beta$3 exprimés au niveau de la membrane des ostéoclastes sont à la base du processus d'adhésion/résorption, contribuent à l'organisation du cytosquelette cellulaire, et sont impliqués dans l'ostéoporose (Ross et al., J. Biol. Chem., 1987, 262, 7703).

Les récepteurs $\alpha$v$\beta$3 exprimés au niveau des cellules du muscle lisse de l'aorte, stimulent leur migration vers la neointima, ce qui entraîne la formation de l'athérosclérose et la survenue de resténose post-angioplastique (Brown et al, cardiovascular Res. (1994), 28, 1815).

Les cellules endothéliales secrètent des facteurs de croissance qui sont mitogènes pour l'endothélium et peuvent contribuer à la formation de nouveaux vaisseaux sanguins (Angiogenèse). La stimulation angiogénique provoque la formation de nouveaux vaisseaux sanguins.

Les antagonistes de l'intégrine $\alpha$v$\beta$3 peuvent ainsi entraîner une régression des tumeurs cancéreuses en induisant l'apoptose des vaisseaux sanguins angiogéniques. (Brook et al. Cell (1994) 79, 1157).

Les ligands naturels de l'intégrine $\alpha$v$\beta$3 contiennent tous le motif RGD (Arg-Gly-Asp). Les peptides contenant ce motif RGD ainsi que des anticorps anti $\alpha$v$\beta$3 sont connus pour leur capacité d'inhibition de la résorption de la dentine, d'empêchement de l'adhésion des ostéoclastes sur les matrices minéralisées (Horton et al. Exp. Cell. Res. (1991), 195, 368).

Le peptide Echistatine isolé du venin de serpent contenant également un motif RGD est décrit comme inhibiteur de l'adhésion des ostéoclastes à l'os, et est donc un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1441).

Les composés de formule (I) ainsi que leurs sels d'addition et leurs esters pharmaceutiquement acceptables peuvent posséder notamment une affinité vis-à-vis du récepteur de la vitronectine $\alpha$v$\beta$3 ou vis-à-vis d'autres intégrines ayant pour ligand la vitronectine ($\alpha$v$\beta$1, $\alpha$v$\beta$5, $\alpha$2b$\beta$3) en inhibant la liaison à leur ligand naturel.

Cette propriété rend ainsi les composés de l'invention utilisables pour la prévention ou le traitement de maladies dont la pathologie sous-jacente est provoquée par les ligands ou les cellules qui interagissent avec le récepteur de la vitronectine.

Ces composés peuvent posséder également une activité vis-à-vis d'autres intégrines qui interagissent avec leur ligand via la séquence tripeptidique RGD, leur conférant des propriétés pharmacologiques utilisables pour traiter les pathologies associées à ces récepteurs.

Cette activité vis-à-vis des intégrines rend ainsi les composés de l'invention utilisable dans le traitement de nombreuses maladies telles que celles mentionnées plus haut ou dans la revue de Dermot Cox DN&P 8(4) Mai 1995,

197-205 dont le contenu est intégré dans la présente demande.

L'invention a donc pour objet les composés de formule (I') à titre de médicaments, ainsi que leurs sels d'addition ou leurs esters pharmaceutiquement acceptables.

Parmi les médicaments de l'invention, on peut citer particulièrement les composés décrits dans la partie expérimentale.

Parmi ces produits, l'invention a plus particulièrement pour objet, à titre de médicaments, les composés de formule (I') listés précédemment.

La posologie varie en fonction de l'affection à traiter et de la voie d'administration : elle peut varier par exemple de 1 mg à 1000 mg par jour chez l'adulte par voie orale.

L'invention s'étend aux compositions pharmaceutiques renfermant comme principe actif au moins un médicament tel que défini ci-dessus.

Les composés de formule (I') sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, de nanosphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II), dans laquelle le radical hydroxy est en position 10, $R'_2$ en position 8 et $R'_3$ en position 9, représentent un groupement O-(Alk) sont préparés selon la méthode décrite dans la demande de brevet européen n° 0729933 et dans la demande de brevet international WO 97/34865 (préparation 2).

Les deux autres isomères de position peuvent être préparés de la manière suivante :

On soumet un composé de formule (IIA) :

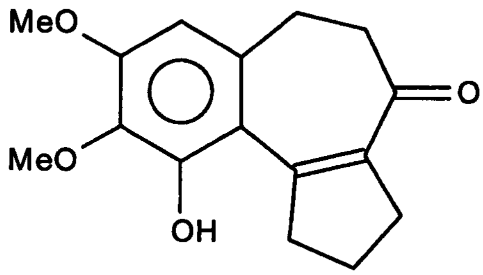

(IIA)

à l'action d'un réactif de déalkylation, afin d'obtenir le composé de formule (IIB) :

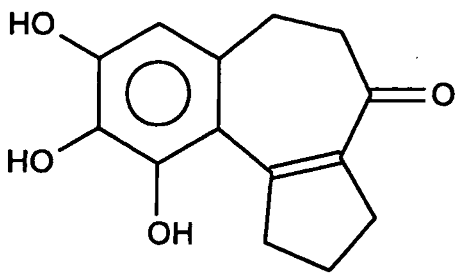

(IIB)

composé de formule (IIB) que l'on soumet :

soit à l'action d'un réactif de protection des diols en milieu basique, afin d'obtenir sélectivement le produit de formule (IIC) :

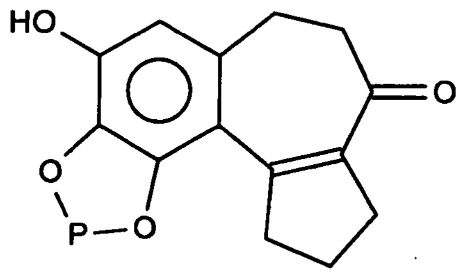

(IIC)

dans laquelle P représente le reste d'un réactif de protection des diols,
que l'on soumet successivement à l'action d'un réactif de protection du phénol, d'un réactif de déprotection des diols,
d'un agent d'alkylation puis d'un agent de déprotection du phénol afin d'obtenir le composé de formule (IID) correspondant au produit de formule (II) trisubstitué avec OH en position 8 :

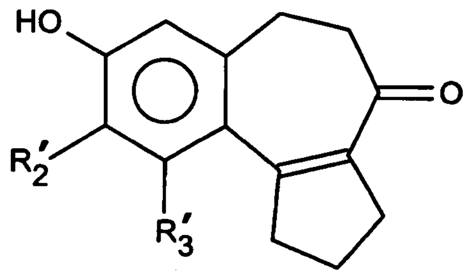

(IID)

soit à l'action successivement d'un agent de protection du phénol, d'un agent d'alkylation puis d'un agent de déprotection afin d'obtenir le composé de formule (IIE) correspondant au produit de formule (II) trisubstitué avec OH en position 9

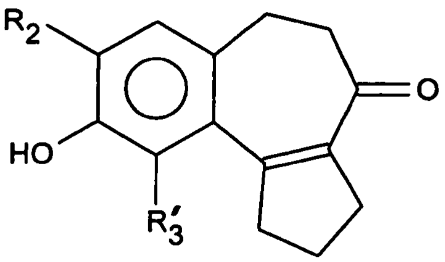

(IIE)

Par réactif de déalkylation, on entend de préférence des agents tels que le tribromure de bore ou le chlorure d'aluminium.

Le réactif de protection des diols que l'on fait réagir sur les produits de formule (IIB) peut être un dérivé du bore tel que l'acide borique, un borate de trialkyle, par exemple de triméthyle ou de triéthyle, ou encore le borax.

Par agent de protection du phénol, on entend notamment un halogénure tel que le chlorure ou le bromure de mésyle ou de tosyle ou encore un dérivé benzylé tel que le tosylate ou le mésylate de benzyle.

Par réactif de déprotection des diols, on entend notamment un acide fort tel que l'acide chlorhydrique, l'acide sulfurique ou bien l'acide paratoluène sulfonique ou encore un oxydant, par exemple l'eau oxygénée, dans le cas d'une protection par un dérivé du bore.

Par agent d'alkylation, on entend tout agent classique connu de l'homme du métier pour alkyler les phénols. On peut citer, par exemple un halogénure d'alkyle tel que le chlorure de méthyle ou d'éthyle, un sulfate d'alkyle tel que le sulfate de méthyle ou d'éthyle, ou encore le diazométhane.

Par agent de déprotection, on entend une base telle que la soude, la potasse ou encore le carbonate de sodium

ou de potassium.

Les produits de formule (II) monosubstitués, dans lesquels $R'_2$ et $R'_3$ représentent un atome d'hydrogène, sont préparés selon une méthode analogue à celle décrite dans la demande de brevet européen n° 0729933 :

(i) On soumet un composé de formule (a) :

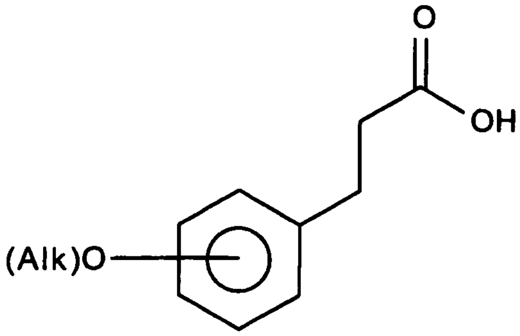

**(a)**

dans laquelle O-(Alk) est en position méta ou para du groupement alkylcarboxylique, (Alk) étant tel que défini précédemment, à l'action d'un agent d'halogénation pour obtenir l'halogénure d'acyle correspondant,

(ii) que l'on soumet à l'action d'un réactif de formule (b) :

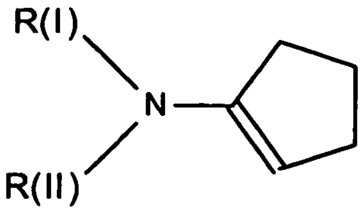

**(b)**

dans laquelle R(I) et R(II), identiques ou différents représentent un groupement alkyle renfermant de 1 à 6 atomes de carbone, ou R(I) et R(II) ensemble avec l'atome d'azote auquel ils sont liés, représentent un hétérocycle à 5 ou 6 chaînons, saturé ou insaturé, renfermant éventuellement un autre hétéroatome choisi parmi O et N,

pour obtenir un composé de formule (c) :

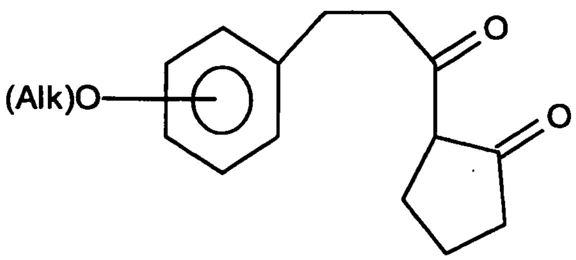

**(c)**

(iii) que l'on soumet à l'action d'un agent d'halogénation pour obtenir un composé de formule (d) :

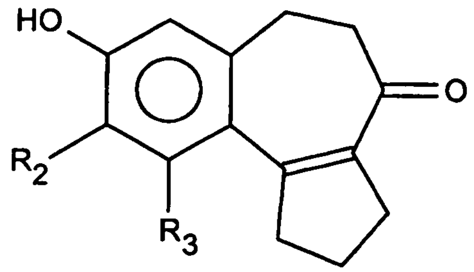

(d)

dans laquelle $Hal_1$ représente un atome d'halogène,

(iv) que l'on soumet à l'action d'un acide de Lewis, pour obtenir un composé de formule (e) :

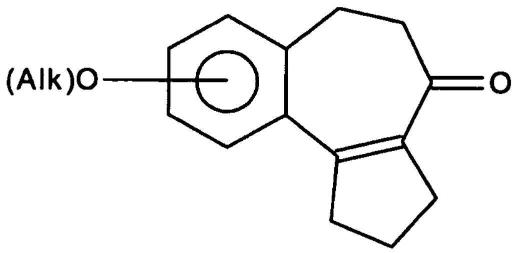

(e)

(v) que l'on soumet à un réactif de déalkylation afin d'obtenir le produit de formule (IIF) correspondant au produit de formule (II) monosubstitué attendu :

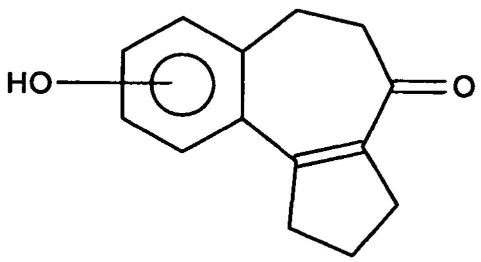

(IIF)

Les produits de formule (II) disubstitués, dans laquelle $R'_2$ représente O-(Alk), $R'_3$ est un atome d'hydrogène et OH et $R'_2$ étant en position 8, 9 ou 10, sont préparés selon la méthode telle que décrite ci-dessus à partir du composé de formule (a') :

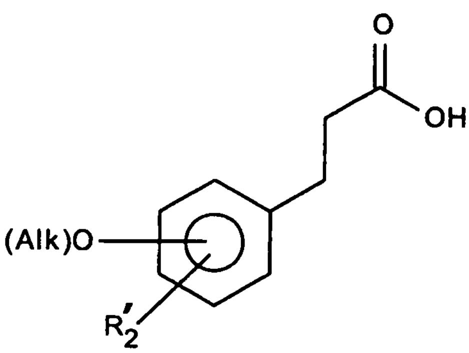

(a')

dans laquelle O-(Alk) et $R'_2$ sont en position méta ou para de la chaîne alkyle carboxylique, $R'_2$ étant un groupement O-(Alk), successivement aux réactions (i), (ii), (iii), (iv) et (v) et on obtient les produits de formule (IIG) correspondant aux produits de formule (II) bisubstitués attendus :

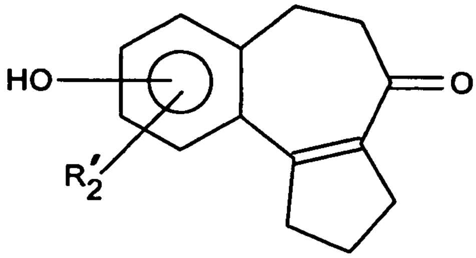

(IIG)

L'agent d'halogénation que l'on fait agir sur le composé de formule (a) ou (a') est par exemple le chlorure de thionyle, le chlorure d'oxalyle ou tout autre agent connu de l'homme du métier pour préparer un halogénure d'acide.
Le réactif de formule (b) est préparé au départ de la cyclopentanone et d'une amine secondaire, par exemple la diéthylamine, la pipéridine, la pipérazine ou, de préférence, la morpholine. On opère en présence d'un catalyseur acide fort, par exemple l'acide paratoluène sulfonique.
L'action de l'énamine de formule (b) sur l'halogénure d'acide est réalisée de préférence en présence d'une amine tertiaire telle que la triéthylamine ou la pyridine.
L'agent d'halogénation que l'on fait réagir sur le composé de formule (c), ou son équivalent disubstitué de formule (c'), peut être par exemple le chlorure de thionyle, le phosgène, l'oxychlorure de phosphore ou, de préférence, le chlorure d'oxalyle.
L'acide de Lewis utilisé pour cycliser le composé de formule (d), ou son équivalent disubstitué de formule (d') est par exemple le chlorure d'aluminium, le tétrachlorure de titane, ou de préférence le chlorure ferrique, ou le tétrachlorure d'étain. La réaction, comme celles qui précèdent, peut être conduite, par exemple, dans un solvant halogéné tel que le chlorure de méthylène, le chloroforme ou le dichloroéthane.
Le réactif de déalkylation du composé de formule (e), ou son équivalent disubstitué de formule (e') afin d'obtenir les phénols correspondant est de préférence le chlorure d'aluminium ou le tribromure de bore.
Les produits de formule (II) dans laquelle il y a une double liaison en position 1-2 sont préparés selon les méthodes connues de l'homme du métier et notamment selon la méthode décrite dans la demande de brevet européen n° 0729933, c'est-à-dire par déshydratation ou désalcoxylation en milieu acide anhydre.
Les produits de formule (II) dans laquelle la jonction entre le cycle à 5 et le cycle à 7 est saturée sont préparés selon les méthodes classiques d'hydrogénation notamment en présence de palladium sur charbon de la double liaison correspondante.
L'invention a également pour objet, à titre de produits intermédiaires, les produits de formule (IIIa), (IIIb), (IIIc) et (IIId).
Les exemples suivants illustrent l'invention sans toutefois la limiter.

EXEMPLE 1 : 3-[[[4-[(4,5-dihydro-1H-imidazol-2-yl)hydrazono]-9,10-diméthoxy-1,2,3,4,5,6-hexahydro-8-benz[e]azulenyl]-carbonyl]amino]-N-[(phénylméthoxy)carbonyl]-DL-alanine

Stade A : 9,10-diméthoxy-1,2,3,4,5,6-hexahydro-4-oxobenz[e]azulene-8-carboxylate de méthyle

Activation (formation du triflate) puis carbonylation

A une solution à 0-5°C de 2,74 g de 9,10-diméthoxy-8-hydroxy-2,3,5,6-tétrahydro-benz[e]azulen-4(1H)-one préparé comme indiqué à la préparation 3 de la demande de brevet WO 97/34865, dans 20 ml de pyridine, on ajoute 2,7 ml d'anhydride triflique et agite 3 heures à cette température. On verse dans l'eau, extrait, sèche et évapore sous pression réduite jusqu'à obtention de 4,58 g de produit brut.
On mélange 1,374 g du triflate obtenu plus haut, 84 mg de $Pd(OAc)_2$, 156 mg de 1,1'-bis(diphénylphosphino) ferrocene (Fluka), 0,82 ml de triéthylamine, 9,1 ml de diméthylsufoxyde, 4,1 ml de 1,2-dichloroéthane, place sous atmosphère de CO et porte à 80°C pendant 5 heures puis 16 heures à température ambiante. On verse dans l'eau, extrait, sèche et purifie par chromatographie en éluant avec le mélange cyclohexane/acétate d'éthyle.
On obtient 420 mg de produit pur attendu.

| | |
|---|---|
| IR ($CHCl_3$) | |
| C=O | 1723 $cm^{-1}$ ester méthylique |
| | 1650 $cm^{-1}$ cétone conjuguée |
| C=C | 1587 $cm^{-1}$, 1550 $cm^{-1}$ |
| + aromatique | |

Stade B : 3-[[(9,10-diméthoxy-1,2,3,4,5,6-hexahydro4-oxo-8-benz[e]azulenyl]carbonyl]amino]-N-[(phénylméthoxy)carbonyl]-DL-alaninate de méthyle

Saponification (formation de l'acide) puis amidification

A 382 mg de l'ester obtenu au stade précédent dans 5 ml de méthanol, on ajoute 2,4 ml de soude 1N et agite 1 heure. Après évaporation du méthanol, dilution à l'eau et extraction avec l'acétate d'éthyle, on acidifie jusqu'au pH 1-2 avec de l'acide chlorhydrique 1N, extrait à l'acétate d'éthyle et sèche. On obtient 348 mg de produit brut.

On agite à température ambiante 1 nuit sous atmosphère inerte 338 mg de l'acide obtenu plus haut, 10 ml de diméthylformamide, 540 mg O-(benzotriazol-1-yl)-N,N,N',N'-tétraméthyluronium tétrafluoroborate (Fluka), 0,62 ml de diisopropyléthylamine et 330 mg d'ester méthylique de l'acide -N-$\alpha$-($CO_2$-$CH_2$-phényl)-$\alpha$,$\beta$-diaminopropionique, obtenu selon le procédé décrit par Tamura Noribazu Chem. Pharm. Bull. 39 (5), 1199 (1991). Après dilution à l'acétate d'éthyle, lavage, séchage et évaporation sous pression réduite on obtient 622 mg de produit attendu.

| | |
|---|---|
| IR ($CBCl_3$) | |
| =C-NH | 3423 $cm^{-1}$, 3376 $cm^{-1}$ |
| C=O | 1721 $cm^{-1}$, 1672 (ep), 1655 (max) $cm^{-1}$ |
| C=C + | 1625 $cm^{-1}$ |
| amide II | 1600, 1587, 1528, 1512 $cm^{-1}$ |
| NR/$NH_2$ | 1534 $cm^{-1}$, 1491 $cm^{-1}$ |

Stade C : 3-[[[4-[(4,5-dihydro-1H-imidazol-2-yl)hydrazono]-9,10-diméthoxy-1,2,3,4,5,6-hexahydro-8-benz[e]azulenyl]-carbonyl]amino]-N-[(phénylméthoxy)carbonyl]-DL-alaninate de méthyle

Introduction de G

On mélange 16 heures à 120°C, 300 mg de l'amide obtenue au stade B, 304 mg de bromhydrate de 2-hydrazino-2-imidazoline et 2,5 ml de butanol. On évapore sous pression réduite jusqu'à obtention de 600 mg de produit brut que l'on purifie par chromatographie en éluant avec le mélange dichlorométhane/méthanol/hydroxyde d'ammonium 90/10/2. On obtient 293 mg de produit pur attendu.

| | |
|---|---|
| IR ($CBCl_3$) | |
| -NH | 3444, 3320 $cm^{-1}$ |
| C=O | 1742 $cm^{-1}$ (ep), 1721 (max) $cm^{-1}$ |
| C=C + | 1625 $cm^{-1}$ |
| C=N | 1648 (ep), 1625, 1512 $cm^{-1}$ (max, F) |
| Système | 1595 (ep), 1530, 1510 $cm^{-1}$ |
| conjugué + aromatique | |

Stade D : 3-[[[4-[(4,5-dihydro-1H-imidazol-2-yl)hydrazono]-9,10-diméthoxy-1,2,3,4,5,6-hexahydro-8-benz[e]azulenyl]-carbonyl]amino]-N-[(phénylméthoxy)carbonyl]-DL-alanine Saponification de l'ester méthylique

On agite à température ambiante pendant 1 heure 30, 262 mg de l'ester obtenu au stade C, 3 ml de méthanol et 0,45 ml de soude 2N.

Après dilution dans 5 ml d'eau, extraction à l'acétate d'éthyle, acidification de la phase aqueuse jusqu'à pH 6 avec de l'acide chlorhydrique 1N, filtration et séchage, on obtient 166 mg de produit pur attendu.

| RMN ($CDCl_3$) | |
| --- | --- |
| 1,86 | CE, en position 2 |
| 2,02 | |
| 2,70 à 3,85 | =C-C$\underline{H}_2$ et =N-C$\underline{H}_2$ |
| 3,61 (s), 3,68 (s), 3,75 (s) | Ph-O-C$\underline{H}_3$ |
| 4,02 (m), 4,10 | -C(O)-C$\underline{H}$-NHC(O)- |
| 5,04 (AB) | $CO_2$-C$\underline{H}_2$-Ph |
| 7,60 | H aromatique |
| 7,48 (s), 7,50 (s) | Ph-H, H en position 7 |
| 7,88 | H mobile |
| 8,44 | CON$\underline{H}$-$CH_2$ |

EXEMPLE 2 : 3-[[[9,10-diméthoxy-1,2,3,4,5,6-hexahydro-4-[(1,4,5,6-tétrahydropyrimidin-2-yl)]-hydrazonol-8-benz[e] azulenyl]carbonyl]amino]-N-[(phénylméthoxy)carbonyl]-DL-alanine

On opère comme à l'exemple 1, stades A, B, C et D mais en utilisant comme aminoguanidine cyclique le bromhydrate de 2-hydrazino-1,4,5,6-tétrahydropyrimidine (Acros)

| RMN ($CDCl_3$) | |
| --- | --- |
| 1,87 | $CH_2$ centraux |
| 2,40 à 3,00 | =C-C$\underline{H}_2$ |
| 3,40-3,77 | =C-N-C$\underline{H}_2$ |
| 4,20 (m) | C(O)-C$\underline{H}$-NHC(O) |
| 3,71 (s) | Ph-OMe |
| 5,04 [AB] | $CO_2$-C$\underline{H}_2$-Ph |
| ≈ 7,30 (m), 7,44 (s1) 7,51 (m) | H aromatique |
| 8,30 à 8,43 | H mobile |

EXEMPLE 3 : Acide bêta-[[[4-[(4,5-dihydro-1H-imidazol-2-yl) hydrazono]-1,2,3,4,5,6-hexahydro-8-benz[e]azulenyl]carbonyl]-amino]-3-pyridine-3-yl-propanoique

Stade A : 1,2,3,4,5,6-hexahydro-4-oxo-benz[e]azulene-8-carboxylate de méthyle

Activation (formation du triflate) puis carbonylation

On opère comme à l'exemple 1, stade A mais à partir de 1,3 g de 8-hydroxy-2,3,5,6-tétrahydro-benz[e]azulen-4(1H)-one préparé comme indiqué à la préparation 7 de la demande de brevet WO 97/34865. On obtient 678 mg de produit attendu
(F = 126-128°C).

| IR ($CHCl_3$) | |
| --- | --- |
| C=O | 1719 $cm^{-1}$, 1645 $cm^{-1}$ |
| C=C + aromatiques | 1608, 1594, 1560, 1497 $cm^{-1}$ |
| $CO_2Me$ | 1438 $cm^{-1}$ |

Stade B : 3-[[(9,10-diméthoxy-1,2,3,4,5,6-hexahydro-4-oxo-8-benz[e]azulenyl]carbonyl]-amino]-3-pyridine-3-yl-propanoate d'éthyle

Saponification de l'ester puis amidification

On opère comme à l'exemple 1, stade B mais à partir de 670 mg du produit obtenu au stade précédent et en

utilisant 0,188 g de chlorhydrate de 3-amino-3-pyridin-3-yl-propanoate d'éthyle, obtenu selon le procédé décrit par Secor J. Org. Chem. NY, 3136-8 (1979).

On obtient 271,2 mg de produit pur attendu.

Stade C : Acide bêta-[[[4-[(4,5-dihydro-1H-imidazol-2-yl) hydrazono]-1,2,3,4,5,6-hexahydro-8-benz[e]azulenyl]carbonyl]-amino]-3-pyridin-3-yl-propanoate d'éthyle

Introduction de G

On opère comme au stade C de l'exemple 1, à partir de 271,2 mg du produit obtenu au stade précédent. On obtient 93,7 mg de produit attendu.

| | |
|---|---|
| IR ($CECl_3$) | |
| NH | 3452 $cm^{-1}$ |
| C=O | 1723 $cm^{-1}$ |
| C=O, C=N, C=C, aromatiques | 1644, 1622 (Max, F), 1547, 1514, 1487 $cm^{-1}$ |

Stade D : Acide bêta-[[[4-[(4,5-dihydro-1B-imidazol-2-yl) hydrazono]-1,2,3,4,5,6-hexahydro-8-benz[e]azulenyl]carbonyl]-aminol-3-pyridin-3-yl-propanoique

Saponification de l'ester

On opère comme au stade D de l'exemple 1, à partir de 90 mg du produit obtenu au stade précédent. On obtient 67,6 mg de produit pur attendu. F = 218°C.

| | |
|---|---|
| Rf $CH_2Cl_2$/MeOH/$NH_2OH$ | 40/20/2 = 0,25 |
| 1,85 (m) | $CE_2$ en position 2 |
| 2,65 à 3,01 10 H | =C-$CH_2$ |
| 3,44 (s) | =N-$CH_2$ |
| 5,42 (q) | =C-C<u>H</u>-NHCO |
| 7,33 (m) 2H ; 7,70 (m) 2H, 7,18 (m) $H'_5$ ; 8,46 (dd) $H'_6$ ; 8,60 (d) $H'_2$ aromatique | |
| 8,94 (d) | H mobile CO-N<u>H</u>-CE |

EXEMPLE 4 : Acide bêta-[[[4-[(4,5-dihydro-1H-imidazol-2-yl) hydrazono] 1,2,3,4,5,6-hexahydro-4-oxo-8-benz[e] azulenyl]carbonyl]-amino]-3-(1,3-benzodioxole-5-yl-propanoique

On opère comme à l'exemple 3, stades A, B, C et D mais en utilisant au stade B, le chlorhydrate de 3-amino-3-(1,3-benzodioxol-5-yl)-propanoate d'éthyle, obtenu selon le procédé décrit par A. Zablochi J. Med. Chem. 38(13) 2378-2394 (1995).

| | |
|---|---|
| RMN (DMSO) | |
| 1,83 (m) | $CH_2$ en position 2 |
| 2,60 à 2,95 10 H | =C-$CH_2$ |
| 3,42 | =N-$CH_2$ |
| 5,32 (q) | C(O)-NH-C<u>H</u>(Ph)-$CH_2$ |
| 5,96 (sl) | -O-C<u>$H_2$</u>-O- |
| 6,82 [AB] | $H'_4$ et $H'_5$ |
| 6,99 (sl) | $H'_7$ |
| 7,65 (sl) | H aromatique H en position 7 |
| 7,35 (d) | H aromatique H en position 10 |
| 7,69 (d) | H aromatique H en position 9 |
| 8,78 (d) | =C-N<u>H</u>-CH |

Compositions pharmaceutiques

Comprimés répondant à la formule suivante :

- produit de l'exemple 1 50 mg
- Excipient (talc, amidon, stéarate de magnésium) QS pour un comprimé terminé à 120 mg

Etude pharmacologique des produits de l'invention

1 - Etude par les produits de l'invention du déplacement de la liaison : Vitronectine/récepteur Vitronectine ($\alpha_v\beta_3$)
Protocole :

Des plaques 96 puits MaxiSorp sont coatées une nuit à 4°C, avec 100 µl de Vitronectine humaine (cf Yatohgo et al. Cell., Structure and fraction 13 : 281-292 (1988)) à 2 µg/ml, (Dilution en tampon de coating).
Le lendemain, les puits sont vidés et les ligands (Vitronectine) sont ensuite fixés (voir tampon de fixation) pendant 1H à température ambiante sous agitation douce.
Les puits sont lavés six fois (voir tampon de lavage), puis on ajoute par puits et dans cet ordre :

- 40 µl de tampon d'incubation,
- 10 µl de la dilution du produit à tester,

(les produits sont dilués dans un mélange 50/50 de DMSO-$H_2O$)

- 50 µl de récepteur $\alpha_v\beta_3$ humain (cf Pytela et al. Methods Enzymol (1987) 144:475) (dilution en tampon d'incubation, à adapter suivant le lot de récepteur et selon le ligand).

Le ligand, le récepteur $\alpha_v\beta_3$ humain et les produits à étudier sont incubés pendant 3 heures à température ambiante sous agitation douce.
Les puits sont à nouveau lavés six fois, puis incubés pendant 2 heures à température ambiante sous agitation douce, en présence de 100 µl d'anticorps 4B12-HRP, anti-récepteur couplé à une peroxydase (l'anticorps 4B12-HRP est dilué en tampon d'incubation. La dilution est à adapter suivant le lot de récepteur).
Les puits sont ensuite lavés six fois avant la mesure de liaison ligand-récepteur faite par l'intermédiaire d'un kit révélateur de peroxydase (TMB Microwell Peroxidase Substrate System Kirkegaard : Réf. cat. 50-76-00).
Ce kit contient un flacon A de substrat (3,3',5,5'-tétraméthylbenzidine à 0,4 g/l) et un flacon B ($H_2O_2$ à 0,02 % en tampon Citrate/Acide citrique). Extemporanément, un volume de A est mélangé à un volume de B, puis le mélange réactionnel est distribué à raison de 100 µl/puits. La réaction enzymatique se développe en 12' pour Vitronectine/$\alpha_v\beta_3$, puis son évolution est stoppée par l'addition de 100 µl d'acide phosphorique 1M.
La densité optique est mesurée à 450 nm.

Tampons :

- tampon de coating : Carbonate 0,05 M, NaOH pH 9,6
- tampon de fixation : PBS contenant 0,5 % de BSA (pH 7,4)
- tampon de lavage : PBS contenant 0,05 % de Tween 20 (pH 7,4)
- tampon d'incubation :
  - 50 mM TRIS pH 7,4
  - 0,5 % BSA
  - 0,05 % Tween 20
  - 1 mM $MnCl_2$
  - 50 µM $CaCl_2$
  - 50 µM $MgCl_2$
  - 100 mM NaCl.

Expression des résultats :

On trace la courbe suivante : le pourcentage de liaison de la vitronectine humaine en fonction du logarithme de la concentration de chaque produit testé.

Pour chaque produit on détermine l'$IC_{50}$ suivant la formule suivante :

$$IC_{50} = (BO + Bmin)/2$$

BO = Maximum de liaison en l'absence de tout produit
Bmin = Minimum de liaison en présence de la concentration la plus élevée de produit.

**RESULTATS :**

| Exemples | Test de compétition binding Vn/VR (($\alpha_v\beta_3$) $IC_{50}$ en $\mu$M |
|---|---|
| EX. 1 | 0,016 |
| EX. 2 | 0,019 |
| EX. 3 | 0,10 |
| EX. 4 | 0,062 |

## Revendications

**1.** Composés de formule générale (I') :

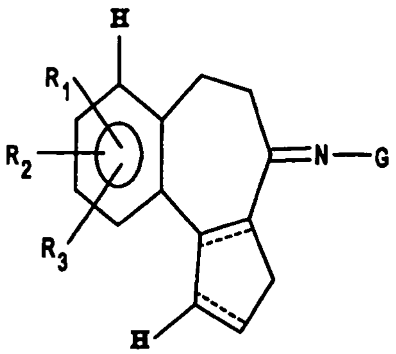

(I')

dans laquelle R'$_1$ représente un groupement -CONH-[A]-[B]-$COR_6$, -[A]- représentant un radical bivalent -$CH_2$-$CH_2$-, substitué par le groupement (Z') ou non substitué, [B] représentant une simple liaison, (Z') représente un atome d'hydrogène, un groupement -NE-$CO_2$-Rc ou Rc, Rc représente un atome d'hydrogène, un radical $(CH_2)_{0-3}$-Ar dans lequel Ar représente un groupement aryle carbocyclique renfermant de 6 à 18 atomes de carbone, un radical $(CB_2)_{0-3}$-Het dans lequel Het représente un radical dérivé d'un hétérocycle aromatique ou non aromatique, saturé ou non saturé, comportant de 1 à 9 atomes de carbone et de 1 à 5 hétéroatomes choisis parmi les atomes d'oxygène, d'azote ou de soufre, un radical $(CB_2)_{0-3}$-Alk dans lequel Alk représente radical dérivé d'un hydrocarbure, non aromatique, linéaire, ramifié ou cyclique, saturé ou insaturé, et comportant de 1 à 12 atomes de carbone, le radical Ar étant non substitué ou bien substitué par -O-$CH_2$-O- lorsque Ar est phényle,

- $R_6$ représente un radical hydroxyle,
- $R'_2$ et $R'_3$ identiques ou différents représentent un atome d'hydrogène ou un radical O-Me
- G représente,

soit un radical de formule G1

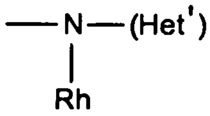

dans lequel Rh est un atome d'hydrogène ou un groupement (Alk) tel que défini précédemment et (Het') est un hétérocycle de formule générale :

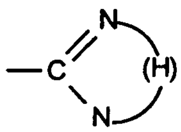

dans lequel (H) forme, avec le motif N=C-NH-, le reste d'un hétérocycle non aromatique, mono ou bicyclique, non saturé, comportant de 1 à 9 atomes de carbone et de 2 à 5 hétéroatomes choisis parmi les atomes d'oxygène, d'azote et de soufre, ce radical pouvant être substitué ou non substitué,

- soit un radical -NRh-C(=X)-NHRc (radical G2), dans lequel X est un atome de soufre, d'oxygène ou NH, Rh et Rc sont tels que définis précédemment,

les traits en pointillés représentent une éventuelle seconde liaison, ainsi que les sels d'addition avec les acides et les bases.

$R'_1$, $R'_2$ et $R'_3$ étant respectivement en position 8, 9 ou 10 du tricycle.

**2.** Composés de formule générale (I') telle que définie à la revendication 1, dans laquelle $R'_1$ représente un groupement -CONH-CH(Z')-$CH_2CO_2H$ ou -CONH-$CH_2$-CH(Z')-$CO_2H$,Z' étant tel que défini à la revendication 1 ainsi que les sels d'addition avec les acides, les bases et les esters.

**3.** Composés de formule générale (I') telle que définie à la revendication 1 ou 2, dans laquelle (Z') est un groupement aryle ou hétéroaryle non substitué, ou un groupement phényle substitué par -O-$CH_2$-O-, ainsi que les sels d'addition avec les acides, les bases et les esters.

**4.** Composés de formule générale (I') telle que définie à l'une des revendications 1 à 3, dans laquelle G est un groupement G2 de formule générale -NE-C(=NH)-NERc, Rc étant tel que défini à la revendication 1, ainsi que les sels d'addition avec les acides, les bases et les esters.

**5.** Composés de formule générale (I') telle que définie à la revendication 4, dans laquelle Rc est un atome d'hydrogène, ainsi que les sels d'addition avec les acides, les bases et les esters.

**6.** Composés de formule générale (I') telle que définie à l'une des revendications 1 à 3, dans laquelle G est un groupement NH-(Het'), (Het') étant tel que défini à la revendication 1.

**7.** Composés de formule générale (I') telle que définie à la revendication 6,
dans laquelle G est le groupement

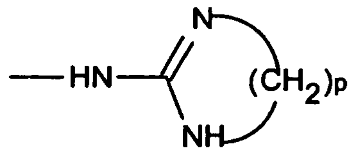

p étant un entier égal à 2, 3 ou 4, ainsi que les sels d'addition avec les acides, les bases et les esters.

**8.** Composés de formule (I') telle que définie à la revendication 1 dont les noms suivent :

- 3-[[[4-[(4,5-dihydro-1H-imidazol-2-yl)hydrazono]-9,10-diméthoxy-1,2,3,4,5,6-hexahydro-8-benz[e]azulenyl]-carbonyl]amiao]-N-[(phénylméthoxy)carbonyl]-DL-alanine
- 3-[[[9,10-diméthoxy-1,2,3,4,5,6-hexahydro-4-[(1,4,5,6-tétrahydropyrimidin-2-yl)]-hydrazono]-8-benz[e] azulenyl]carbonyl]amino]-N-[(ph2nylméthoxy)carbonyl]-DL-alanine
- Acide bêta-[[[4-[(4,5-dihydro-1H-imidazol-2-yl) hydrazono]-1,2,3,4,5,6-hexahydro-8-benz[e]azulenyl]carbonyl]-amino]-3-pyridine-3-yl-propanoique
- Acide bêta-[[[4-[(4,5-dihydro-1H-imidazo-2-yl) hydrazono]-1,2,3,4,5,6-hexahydro-4-oxo-8-benz[e] azulenyl] carbonyl]-amino]-3-(1,3-benzodioxole-5-yl-propanoique.

**9.** Procédé de préparation des composés de formule (I') telle que définie à la revendication 1, comprenant les étapes suivantes :

a) action d'un agent activant de la fonction alcool puis réaction de carbonylation des composés de formule (II) :

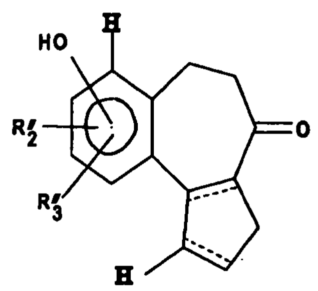

**(II)**

dans laquelle $R'_2$, $R'_3$ sont tels que décrits à la revendication 1, à l'exception de la valeur hydroxyle, afin d'obtenir l'ester de formule (IIIa) :

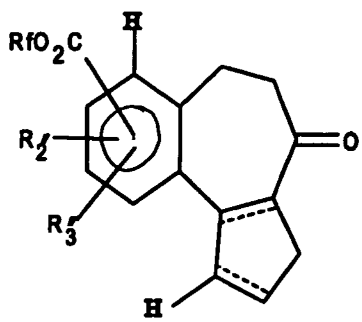

**(IIIa)**

Rf étant un radical alkyle renfermant de 1 à 4 atomes de carbone,

b) saponification de l'ester de formule (IIIa) afin d'obtenir l'acide correspondant de formule (IIIb) :

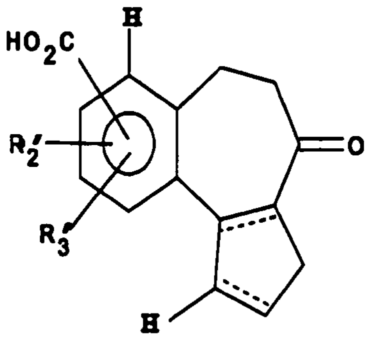

(IIIb)

c) réaction d'amidification de l'acide de formule (IIIb) par action d'un composé de formule (F1), le cas échéant sous forme de sel :

$H_2N$-[A]-[B]-$COR_6$ (F1)

[A], [B] et $R_6$ étant tels que définis à la revendication 1, [A] ou [B] pouvant également représenter le groupement -CH-NHP, P étant un groupement protecteur de la fonction amine, afin d'obtenir un composé de formule (IIIc) :

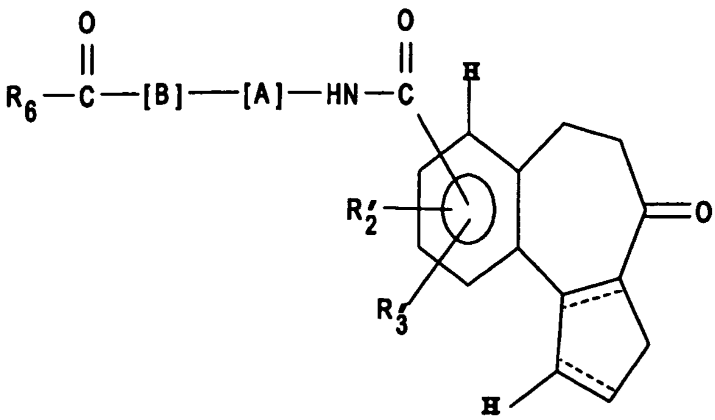

(IIIc)

d) action sur le composé de formule (IIIc) d'un composé de formule (F2) :

G-$NH_2$ (F2)

G étant tel que défini à la revendication 1, afin d'obtenir un composé de formule (I),
e) composé de formule (I) que l'on soumet le cas échéant dans un ordre approprié :

- action d'une base ou d'un acide afin de cliver l'ester et obtenir l'acide correspondant,
- à l'action d'un réactif de déalkylation,
- à l'action d'un agent de déprotection de la fonction NH-P en bêta de CO-$R_6$ lorsque [A] ou [B] représente le groupe CH-NHP,
- à la formation du groupement NH-$CO_2R_c$ à partir de l'amine correspondante,
- à l'action d'un acide ou d'une base afin d'obtenir les sels correspondants ou
- à l'action d'un agent d'estérification afin d'obtenir les esters correspondants.

**10.** Procédé selon la revendication 9 de formation des composés de formule (I') telle que définie à la revendication 1, **caractérisé en ce que** l'on soumet préalablement un composé de formule (II) à l'action d'un composé de formule (F2) afin d'obtenir un composé de formule (IIId) :

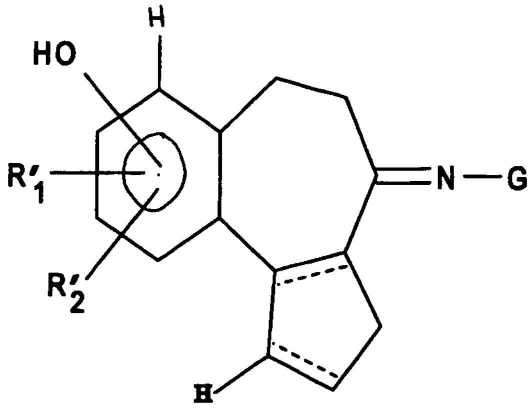

(IIId)

composé de formule (IIId) qui est mis en oeuvre ensuite dans les réactions telles que décrites aux étapes a), b), c) et le cas échéant e).

**11.** A titre de médicament, les composés de formule (I') telle que définie à l'une quelconque des revendications 1 à 7, ainsi que leurs sels d'addition et leurs esters pharmaceutiquement acceptables.

**12.** A titre de médicament, les composés de formule (I') tels que définis à la revendication 8.

**13.** Les compositions pharmaceutiques renfermant comme principe actif au moins l'un des médicaments définis à la revendication 11 ou 12.

**14.** A titre de produits intermédiaires nouveaux, les composés de formules générales (IIIa), (IIIb), (IIIc) et (IIId) telles que définies à la revendication 9 ou 10.

**Claims**

**1.** Compounds of general formula (I'):

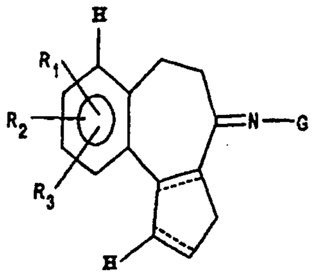

(I')

in which R'$_1$ represents a group -CONH- [A] - [B] -COR$_6$,
-[A]- representing a bivalent radical -$CH_2$-$CH_2$-, substituted with the group (Z') or unsubstituted,
[B] representing a single bond,
(Z') represents a hydrogen atom, a group -NH-$CO_2$-Rc or Rc,
Rc represents a hydrogen atom, a radical $(CH_2)_{0-3}$-Ar in which Ar represents a carbocyclic aryl group containing from 6 to 18 carbon atoms, a radical $(CH_2)_{0-3}$-Het in which Het represents a radical derived from a saturated or unsaturated, aromatic or non-aromatic heterocycle comprising from 1 to 9 carbon atoms and from 1 to 5 heteroatoms chosen from oxygen, nitrogen or sulphur atoms, a radical $(CH_2)_{0-3}$-Alk in which Alk represents a radical derived from a saturated or unsaturated, linear, branched or cyclic, non-aromatic hydrocarbon, and comprising from 1 to 12 carbon atoms, the radical Ar being unsubstituted or substituted with -O-$CH_2$-O-when Ar is phenyl,

- R$_6$ represents a hydroxyl radical,

- $R'_2$ and $R'_3$, which are identical or different, represent a hydrogen atom or a radical O-Me
- G represents,
either a radical of formula G1

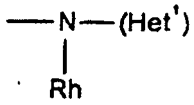

in which Rh is a hydrogen atom or a group (Alk) as defined above and (Het') is a heterocycle of general formula:

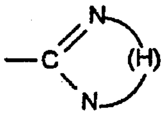

in which (H) forms, with the motif N=C-NH-, the residue of an unsaturated, mono- or bicyclic, non-aromatic heterocycle comprising from 1 to 9 carbon atoms and from 2 to 5 heteroatoms chosen from oxygen, nitrogen and sulphur atoms, it being possible for this radical to be substituted or unsubstituted,
- or a radical -NRh-C(=X)-NHRc (radical G2), in which X is a sulphur, oxygen or NH atom, Rh and Rc are as defined above,

the dotted lines represent an optional second bond, and the addition salts with acids and bases,
$R'_1$, $R'_2$ and $R'_3$ being at the 8, 9 or 10 position of the tricycle, respectively.

**2.** Compounds of general formula (I') as defined in Claim 1, in which $R'_1$ represents a group -CONH-CH(Z')-$CH_2CO_2H$ or -CONH-$CH_2$-CH(Z')-$CO_2H$, Z' being as defined in Claim 1 and the addition salts with acids, bases and esters.

**3.** Compounds of general formula (I') as defined in Claim 1 or 2, in which (Z') is an unsubstituted aryl or heteroaryl group, or a phenyl group substituted with -O-$CH_2$-O-, and the addition salts with acids, bases and esters.

**4.** Compounds of general formula (I') as defined in one of Claims 1 to 3, in which G is a group G2 of general formula -NH-C(=NH)-NHRc, Rc being as defined in Claim 1, and the addition salts with acids, bases and esters.

**5.** Compounds of general formula (I') as defined in Claim 4, in which Rc is a hydrogen atom, and the addition salts with acids, bases and esters.

**6.** Compounds of general formula (I') as defined in one of Claims 1 to 3, in which G is a group NH-(Het'), (Het') being as defined in Claim 1.

**7.** Compounds of general formula (I') as defined in Claim 6,
in which G is the group

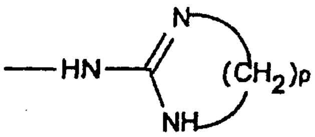

p being an integer equal to 2, 3 or 4, and the addition salts with acids, bases and esters.

**8.** Compounds of formula (I') as defined in Claim 1, whose names are as follows:

- 3-[[[4-[(4,5-dihydro-1H-imidazol-2-yl)hydrazono]-9,10-dimethoxy-1,2,3,4,5,6-hexahydro-8-benz[e]azulenyl]

carbonyl]amino]-N-[(phenylmethoxy)carbonyl]-DL-alanine
- 3-[[[9,10-dimethoxy-1,2,3,4,5,6-hexahydro-4-[(1,4,5,6-tetrahydropyrimidin-2-yl)]-hydrazono]-8-benz[e]azulenyl]carbonyl]amino]-N-[(phenylmethoxy)carbonyl]-DL-alanine
- beta-[[[4-[(4,5-dihydro-1H-imidazol-2-yl)hydrazono]-1,2,3,4,5,6-hexahydro-8-benz[e]azulenyl]carbonylamino]-3-pyridin-3-ylpropanoic acid
- beta-[[[4-[(4,5-dihydro-1H-imidazo-2-yl)hydrazono]-1,2,3,4,5,6-hexahydro-4-oxo-8-benz[e]azulenyl]carbonyl]amino]-3-(1,3-benzodioxol-5-ylpropanoic acid.

**9.** Method for preparing the compounds of formula (I') as defined in Claim 1, comprising the following steps:

a) action of an alcohol functional group activating agent and then carbonylation reaction of the compounds of formula (II):

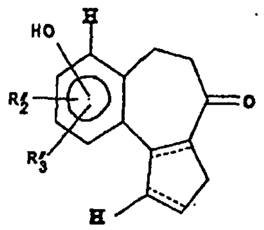

(II)

in which $R'_2$, $R'_3$ are as described in Claim 1, with the exception of the hydroxyl value, so as to obtain the ester of formula (IIIa) :

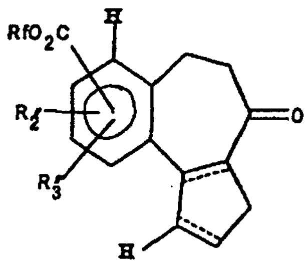

(IIIa)

Rf being an alkyl radical containing from 1 to 4 carbon atoms,
b) saponification of the ester of formula (IIIa) so as to obtain the corresponding acid of formula (IIIb):

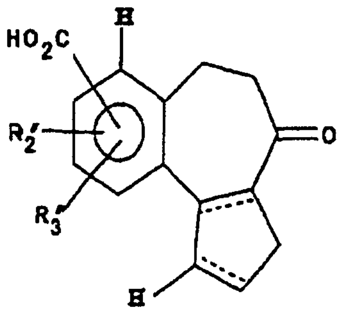

(IIIb)

c) amidation reaction of the acid of formula (IIIb) by the action of a compound of formula (F1), where appropriate in the form of a salt:

$H_2N$-[A]-[B]-$COR_6$ (F1)

[A], [B] and $R_6$ being as defined in Claim 1, it being also possible for [A] or [B] to represent the group -CH-NHP,

P being an amine functional group protecting group so as to obtain a compound of formula (IIIc):

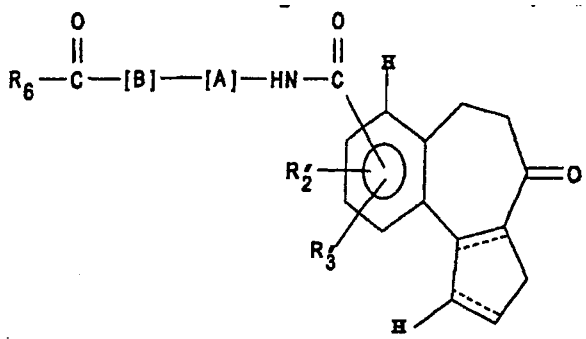

(IIIc)

d) action on the compound of formula (IIIc) of a compound of formula (F2) :

$G\text{-}NH_2$ (F2)

G being as defined in Claim 1, so as to obtain a compound of formula (I),
e) which compound of formula (I) is subjected, where appropriate, in an appropriate order:

- to the action of a base or an acid so as to cleave the ester and obtain the corresponding acid,
- to the action of a dealkylation reagent,
- to the action of an agent for deprotection of the NH-P functional group in beta with respect to $CO\text{-}R_6$ when [A] or [B] represents the group CH-NHP,
- to the formation of the group $NH\text{-}CO_2R_c$ from the corresponding amine,
- to the action of an acid or a base so as to obtain the corresponding salts or
- to the action of an esterifying agent so as to obtain the corresponding esters.

**10.** Method according to Claim 9, for forming the compounds of formula (I') as defined in Claim 1, **characterized in that** a compound of formula (II) is subjected beforehand to the action of a compound of formula (F2) so as to obtain a compound of formula (IIId) :

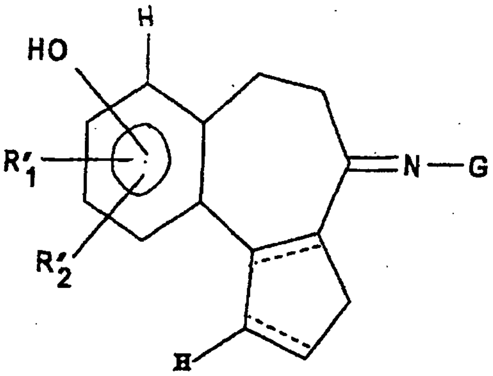

(IIId)

which compound of formula (IIId) is then used in the reactions as described in steps a), b), c) and where appropriate e).

**11.** As a medicament, the compounds of formula (I') as defined in any one of Claims 1 to 7, and their addition salts and their pharmaceutically acceptable esters.

**12.** As a medicament, the compounds of formula (I') as defined in Claim 8.

**13.** Pharmaceutical compositions containing, as active ingredient, at least one of the medicaments defined in Claim 11 or 12.

14. As novel intermediate products, the compounds of general formulae (IIIa), (IIIb), (IIIc) and (IIId) as defined in Claim 9 or 10.

## Patentansprüche

1. Verbindungen mit der allgemeinen Formel (I'):

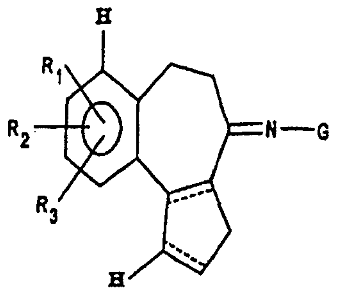

(I')

in welcher $R'_1$ eine Gruppe darstellt aus -CONH-[A]-[B]-$COR_6$, wobei -[A]- ein bivalentes Radikal -$CH_2$-$CH_2$- darstellt, das durch die Gruppe (Z') substituiert wird oder nicht,
[B] eine Einfachverbindung darstellt,
(Z') ein Wasserstoffatom darstellt, sowie eine Gruppe aus -NH-$CO_2$-Rc oder Rc,
Rc ein Wasserstoffatom darstellt und ein Radikal $(CH_2)_{0-3}$-Ar, bei dem Ar eine carbozyklische Arylgruppe darstellt, die 6 bis 18 Kohlenstoffatome umfasst, ein Radikal $(CH_2)_{0-3}$-Het, bei dem Het ein Radikal darstellt, das aus einem aromatischen oder nicht aromatischen gesättigten oder nicht gesättigten Heterozyklus abgeleitet ist, wobei dieser 1 bis 9 Kohlenstoffatome und 1 bis 5 Heteroatome aufweist, die ausgewählt sind aus den Sauerstoffatomen, Stickstoffatomen oder Schwefelatomen, sowie ein Radikal $(CH_2)_{0-3}$-Alk, bei dem Alk ein Radikal darstellt, das aus einem nicht aromatischen, linearen, verzweigten oder zyklischen Kohlenwasserstoff abgeleitet ist, welcher gesättigt ist oder nicht, und welcher 1 bis 12 Kohlenstoffatome aufweist, wobei das Radikal Ar nicht subsitutiert wird oder doch substituiert wird durch -O-$CH_2$-O-, wenn Ar das Phenyl ist,

- $R_6$ ein Hydroxylradikal darstellt,
- $R'_2$ und $R'_3$, die identisch oder voneinander verschieden sind, ein Wasserstoffatom oder ein Radikal O-Me darstellen,
- G entweder ein Radikal der Formel G1 darstellt

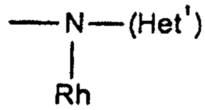

bei der Rh ein Wasserstoffatom oder eine (Alk)-Gruppe ist, wie sie zuvor definiert wurde, und (Het') ein Heterozyklus mit der allgemeinen Formel ist:

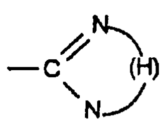

bei der (H) mit dem Motiv N=C-NH- den Rest eines nicht aromatischen, monozyklischen oder bizyklischen Heterozyklus ausbildet, der nicht gesättigt ist und der 1 bis 9 Kohlenstoffatome und 2 bis 5 Heteroatome umfasst,

die ausgewählt sind aus den Sauerstoffatomen, Stickstoffatomen oder Schwefelatomen, wobei dieses Radikal substituiert werden kann oder nicht,

- oder durch ein Radikal -NRh-C(=X)-NHRc (Radikal G2), bei dem X ein Schwefelatom, ein Sauerstoffatom oder NH ist, wobei Rh und Rc derart sind, wie sie zuvor definiert wurden,

und die gepunkteten Linien eine mögliche zweite Bindung darstellen, sowie auch die zusätzlichen Salze mit den Säuren und den Basen,
wobei sich $R'_1$, $R'_2$ und $R'_3$ entsprechend in der Position 8, 9 und 10 des Dreiringes befinden.

2. Verbindungen mit der allgemeinen Formel (I'), wie sie in Anspruch 1 definiert ist, wobei $R'_1$ eine Gruppe aus -CONH-CH(Z')-$CH_2CO_2H$- oder aus -CONH-$CH_2$-CH(Z')-$CO_2$HZ' darstellt, die so beschaffen ist, wie sie in Anspruch 1 definiert ist, sowie auch die zusätzlichen Salze mit den Säuren, Basen und Estern.

3. Verbindungen mit der allgemeinen Formel (I'), wie sie in den Ansprüchen 1 oder 2 definiert ist, bei der (Z') eine Arylgruppe oder eine Heteroarylgruppe ist, die nicht substituiert ist, oder eine Phenylgruppe, die durch -O-$CH_2$-O-substituiert ist, sowie auch die zusätzlichen Salze mit den Säuren, Basen und Estern.

4. Verbindungen mit der allgemeinen Formel (I'), wie sie in einem der Ansprüche 1 bis 3 definiert ist, wobei G eine G2-Gruppe mit der allgemeinen Formel -NH-C(=NH)-NHRc darstellt, wobei Rc derart ist, wie es in Anspruch 1 definiert ist, sowie auch die zusätzlichen Salze mit den Säuren, Basen und Estern.

5. Verbindungen mit der allgemeinen Formel (I'), wie sie in Anspruch 4 definiert ist, bei der Rc ein Wasserstoffatom ist, sowie auch die zusätzlichen Salze mit den Säuren, Basen und Estern.

6. Verbindungen mit der allgemeinen Formel (I'), wie sie in einem der Ansprüche 1 bis 3 definiert ist, wobei G eine NH-(Het')-Gruppe ist und (Het') derart ist, wie es in Anspruch 1 definiert ist.

7. Verbindungen mit der allgemeinen Formel (I'), wie sie in Anspruch 6 definiert ist,
wobei G die Gruppe

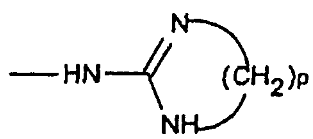

ist und p eine ganze Zahl darstellt, die gleich 2, 3 oder 4 ist, sowie auch die zusätzlichen Salze mit den Säuren, Basen und Estern.

8. Verbindungen mit der allgemeinen Formel (I'), wie sie in Anspruch 1 definiert ist, deren Bezeichnungen folgen:

- 3-[[[4-[(4,5-Dihydro-1H-imidazol-2-yl)hydrazono]-9,10-dimethoxy-1,2,3,4,5,6-hexahydro-8-benz[e]azulenyl]-carbonyl]amino]-N-[(phenylmethoxy)carbonyl]-DL-alanin,
- 3-[[[9,10-Dimethoxy-1,2,3,4,5,6-hexahydro-4-[(1,4,5,6-tetrahydropyrimidin-2-yl)]-hydrazono]-8-benz[e]-azulenyl]carbonyl]amino]-N-[(phenylmethoxy)carbonyl]-DL-alanin,
- Beta-[[[4-[(4,5-Dihydro-1H-imidazol-2-yl)-Hydrazono]-1,2,3,4,5,6-hexahydro-8-benz[e]-azulenyl]carbonyl]-amino]-3-pyridin-3-yl-propansäure,
- Beta-[[[4-[(4,5-Dihydro-1H-imidazo-2-yl)-Hydrazono]-1,2,3,4,5,6-hexahydro-4-oxo-8-benz[e]-azulenyl]carbonyl]-amino]-3-(1,3-benzodioxol-5-yl-propansäure.

9. Verfahren zur Aufbereitung von Verbindungen mit der Formel (I'), wie sie in Anspruch 1 definiert ist, wobei dies die folgenden Schritte umfasst:

a) Reaktion eines Agens in Form der Aktivierung der Alkoholfunktion sowie darauffolgend die Reaktion der Carbonylation der Verbindungen mit der Formel (II):

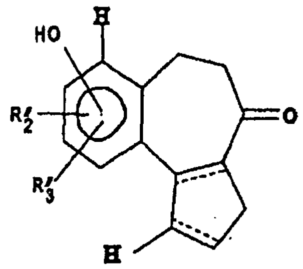

(II)

wobei $R'_2$, $R'_3$ derart sind, wie sie in Anspruch 1 beschrieben sind, ausgenommen der Hydroxylwert, um den Ester mit der Formel (IIIa) zu erhalten:

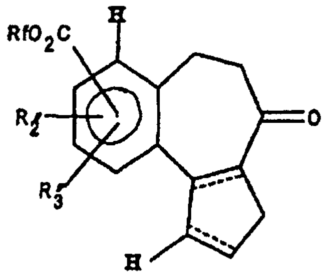

(IIIa)

wobei Rf ein Alkylradikal ist, das 1 bis 4 Kohlenstoffatome umfasst,

b) Verseifung des Esters mit der Formel (IIIa), um eine Säure entsprechend der Formel (IIIb) zu erhalten:

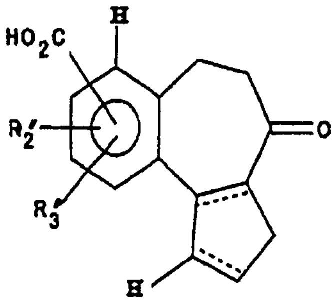

(IIIb)

c) Amidierungsreaktion der Säure mit der Formel (IIIb) durch Reaktion einer Verbindung der Formel (F1), gegebenenfalls in Form eines Salzes:

$H_2N$-[A]-[B]-$COR_6$ (F1)

wobei [A], [B] und $R_6$ derart sind, wie sie in Anspruch 1 definiert sind, wobei [A] oder [B] ebenso die -CH-NHP-Gruppe darstellen können und wobei P eine Schutzgruppe an der Stickstofffunktion darstellt, um eine Verbindung mit der Formel (IIIc) zu erhalten:

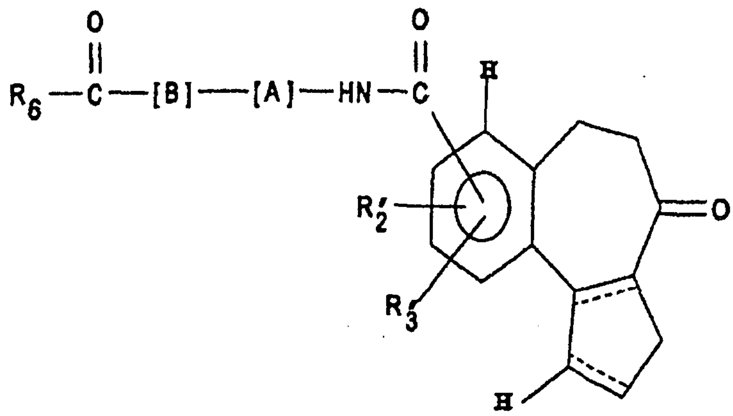

(IIIc)

d) Reagieren der Verbindung mit der Formel (IIIc) durch eine Verbindung mit der Formel (F2):

$G\text{-}NH_2$ (F2)

wobei G derart ist, wie es in Anspruch 1 definiert ist, um eine Verbindung mit der Formel (I) zu erhalten,
e) Verbindung mit der Formel (I), die man gegebenenfalls einer geeigneten Abfolge unterstellt:

- Reaktion einer Base oder einer Säure, um den Ester zu spalten und die entsprechende Säure zu erhalten,
- zur Reaktion einer Reagenz für die Dealkylierung,
- zur Reaktion eines Agens für die Deprotektionierung der beta-NH-P-Funktion von $CO\text{-}R_6$, wenn [A] oder [B] die CH-NHP-Gruppe darstellen,
- zur Bildung der $NH\text{-}CO_2R_6$-Gruppe, ausgehend von dem entsprechenden Amin,
- zur Reaktion einer Säure oder einer Base, um die entsprechenden Salze zu erhalten oder
- zur Reaktion eines Agens für die Esterbildung, um die entsprechenden Ester zu erhalten.

**10.** Verfahren nach Anspruch 9 zur Bildung der Verbindung mit der Formel (I'), wie sie in Anspruch 1 definiert ist, **dadurch gekennzeichnet, dass** man zunächst eine Verbindung mit der Formel (II) der Reaktion mit einer Verbindung mit der Formel (F2) aussetzt, um eine Verbindung mit der Formel (IIId) zu erhalten:

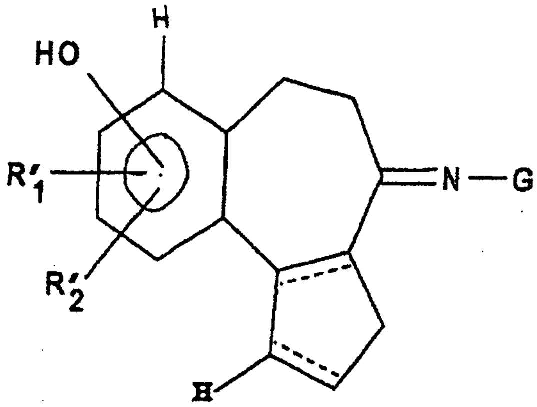

(IIId)

Verbindung mit der Formel (IIId), die in den Reaktionen, wie sie in den Schritten a), b), c) und gegebenenfalls e) beschrieben sind, anschließend hergestellt wird.

**11.** Als Medikament die Verbindungen mit der Formel (I'), wie sie in einem beliebigen der Ansprüche 1 bis 7 definiert ist, sowie auch ihre zusätzlichen Salze und ihre Ester, die pharmazeutisch zugelassen sind.

**12.** Als Medikament die Verbindungen mit der Formel (I'), wie sie in Anspruch 8 definiert sind.

**13.** Pharmazeutische Zusammensetzungen, die als Wirkstoff mindestens eines der Medikamente umfassen, die in

Anspruch 11 oder 12 definiert sind.

**14.** Als neue Zwischenprodukte die Verbindungen mit den allgemeinen Formeln (IIIa), (IIIb), (IIIc) und (IIId), wie sie in Anspruch 9 oder 10 definiert sind.